# EUROPEAN PATENT APPLICATION

(11) **EP 2 100 607 A1**
(43) Date of publication of application: **16.09.2009**
(21) Application number: 07850164.0
(22) Date of filing: 06.12.2007
(51) Int. Cl.: A61K 31/4365, A61K 9/14, A61K 9/16, A61K 9/20, A61K 9/48, A61K 47/32, A61K 47/38, A61P 7/02

(54) **PHARMACEUTICAL COMPOSITION HAVING IMPROVED STORAGE STABILITY**

(30) Priority: 07.12.2006 JP 2006330372
(71) Applicant: Daiichi Sankyo Company, Limited, Chuo-ku Tokyo 103-8426 (JP); Ube Industries, Ltd., Ube City, Yamaguchi 755-8633 (JP)
(72) Inventor: WATANABE, Tomoyuki, Kanagawa 254-0014 (JP); MAEDA, Kazuko, Kanagawa 254-0014 (JP)
(74) Representative: Fairbairn, Angus Chisholm
(86) International application number: PCT/JP2007/073548
(87) International publication number: WO 2008/072532

(57) **Abstract**

Disclosed is a pharmaceutical composition containing a compound represented by the general formula (I) below or a pharmacologically acceptable salt thereof, while having improved storage stability. Specifically disclosed is a pharmaceutical composition containing (A) a compound represented by the general formula (I) below or a pharmacologically acceptable salt thereof, and (B) a water-soluble polymer.

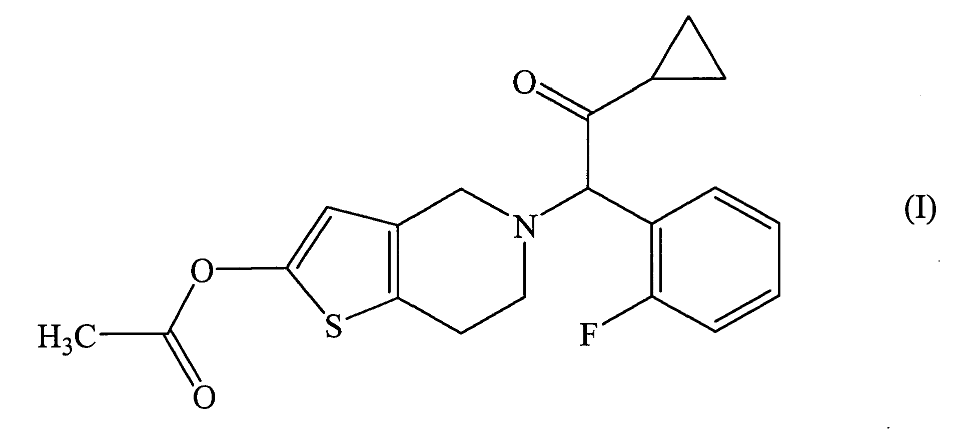

## Description

### [TECHNICAL FIELD]

The present invention relates to a pharmaceutical composition having excellent storage stability, which contains
(A) a compound represented by the following general formula (I): 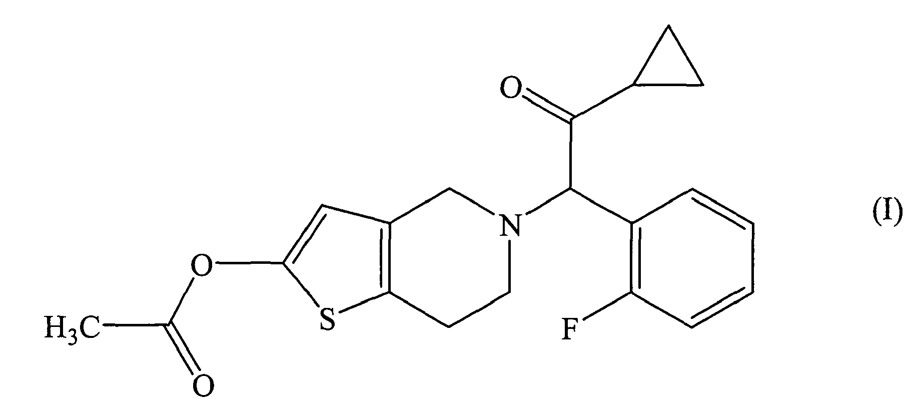 or a pharmacologically acceptable salt thereof, and
(B) a water-soluble polymer.

### [BACKGROUND ART]

The compound represented by the aforementioned general formula (I) or a pharmacologically acceptable salt thereof is known as a compound having platelet aggregation inhibition activity (Patent Document 1 or 2).

Patent Documents 2, 3, 4, 5 and 6 exemplify various kinds of additives that may be used in preparations containing the compound represented by the aforementioned general formula (I) or a pharmacologically acceptable salt thereof, and there is a line which mentions hydroxypropyl cellulose, hydroxypropylmethyl cellulose, polyvinylpyrrolidone and/or polyethylene glycol as such additives. However, they are merely exemplified as some of many types of additives which may be used, and are not specifically used in preparation examples. Further, the aforementioned Patent Documents neither describe nor teach that the storage stability of the compound represented by the aforementioned general formula (I) or a pharmacologically acceptable salt thereof can be improved by including a water-soluble polymer.
[Patent Document 1] Japanese Patent Application (Kokai) No. Hei 6-41139
[Patent Document 2] Japanese Patent Application (Kokai) No. 2002-145883
[Patent Document 3] Japanese Patent Application (Kokai) No. Hei 10-310586
[Patent Document 4] Japanese Patent Application (Kokai) No. 2003-246735
[Patent Document 5] Japanese Patent Application (Kokai) No. 2004-51639
[Patent Document 6] Pamphlet of International Publication WO 2004/098713

### [DISCLOSURE OF THE INVENTION]

### [PROBLEMS TO BE SOLVED BY THE INVENTION]

An object of the present invention is to provide a pharmaceutical composition having excellent storage stability, which contains the compound represented by the aforementioned general formula (I) or a pharmacologically acceptable salt thereof.

### [MEANS FOR SOLVING THE PROBLEMS]

As a result of conducting extensive studies to solve the aforementioned problems, the inventors of the present invention found that a pharmaceutical composition which contains the compound represented by the aforementioned general formula (I) or a pharmacologically acceptable salt thereof can possess excellent storage stability by including a water-soluble polymer, thereby leading to completion of the present invention.

The present invention provides a pharmaceutical composition containing (A) the compound represented by the aforementioned general formula (I) or a pharmacologically acceptable salt thereof and (B) a water-soluble polymer (particularly a composition for prophylaxis or treatment of thrombosis or embolism), use of the compound represented by the aforementioned general formula (I) or a pharmacologically acceptable salt thereof for the production of the pharmaceutical composition (particularly the composition for prophylaxis or treatment of thrombosis or embolism), and a prophylaxis or treatment strategy for a disease (particularly thrombosis or embolism) in which the pharmaceutical composition containing an effective amount of the compound represented by the aforementioned general formula (I) or a pharmacologically acceptable salt thereof is administered to a warm-blooded animal (particularly a human).

That is, the present invention is:
(1) a pharmaceutical composition comprising:
   (A) a compound represented by the following general formula (I): 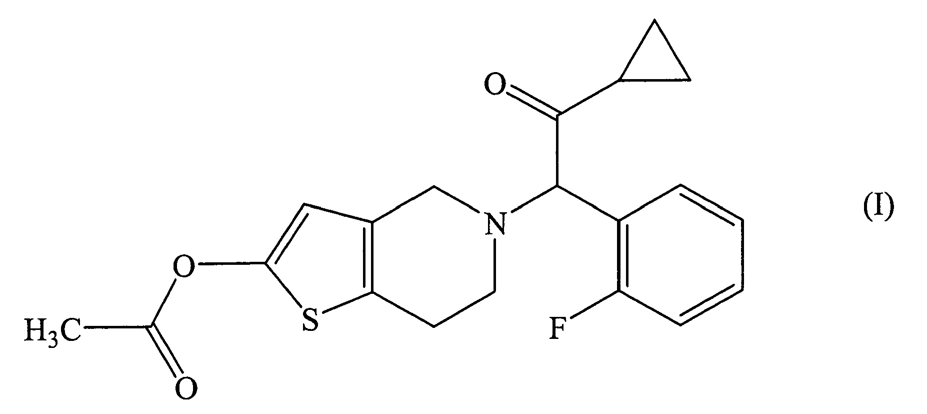 or a pharmacologically acceptable salt thereof; and
   (B) a water-soluble polymer, preferably,
(2) the pharmaceutical composition according to (1), wherein the water-soluble polymer is hydroxypropylmethyl cellulose, hydroxypropyl cellulose or polyvinylpyrrolidone,
(3) the pharmaceutical composition according to (1), wherein the water-soluble polymer is hydroxypropylmethyl cellulose or hydroxypropyl cellulose,
(4) the pharmaceutical composition according to (1), wherein the water-soluble polymer is hydroxypropyl cellulose,
(5) the pharmaceutical composition according to any one of (1) to (4), wherein the amount of the water-soluble polymer formulated is 1.0 to 40.0% by weight with respect to the total amount of the pharmaceutical composition,
(6) the pharmaceutical composition according to any one of (1) to (4), wherein the amount of the water-soluble polymer formulated is 2.5 to 20.0 % by weight with respect to the total amount of the pharmaceutical composition,
(7) the pharmaceutical composition according to any one of (1) to (6), wherein the compound represented by the general formula (I) or a pharmacologically acceptable salt thereof is a compound represented by the following formula (Ia): 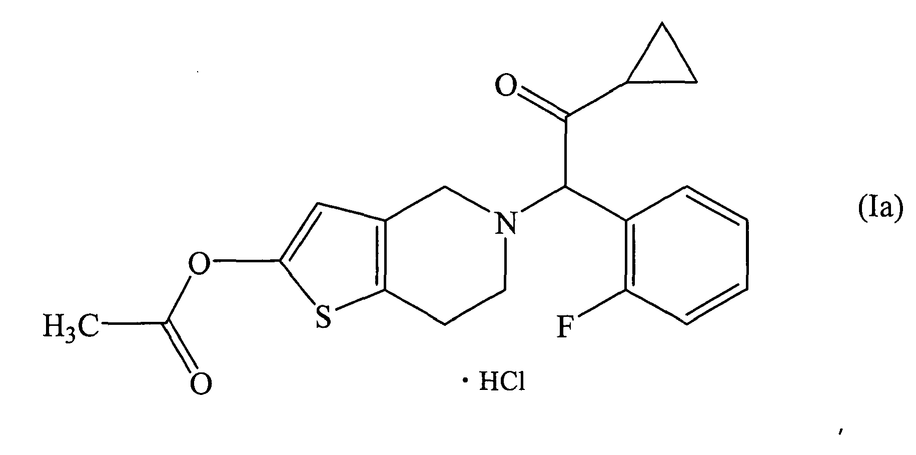
(8) the pharmaceutical composition according to any one of (1) to (7), wherein the composition is in the form of powders, fine granules, granules, capsules or tablets, and
(9) the pharmaceutical composition according to any one of (1) to (7), wherein the composition is in the form of tablets.

### [EFFECT OF THE INVENTION]

According to the present invention, a pharmaceutical composition having excellent storage stability can be provided, which contains (A) the compound represented by the aforementioned general formula (I) or a pharmacologically acceptable salt thereof and (B) a water-soluble polymer.

The pharmaceutical composition of the present invention is, for example, useful for the treatment and/or prophylaxis of thrombosis or embolism (preferably thrombosis) and the like (preferably is a drug for the treatment and/or prophylaxis of thrombosis).

### [BEST MODE FOR CARRYING OUT THE INVENTION]

The compound represented by the following general formula (I) : 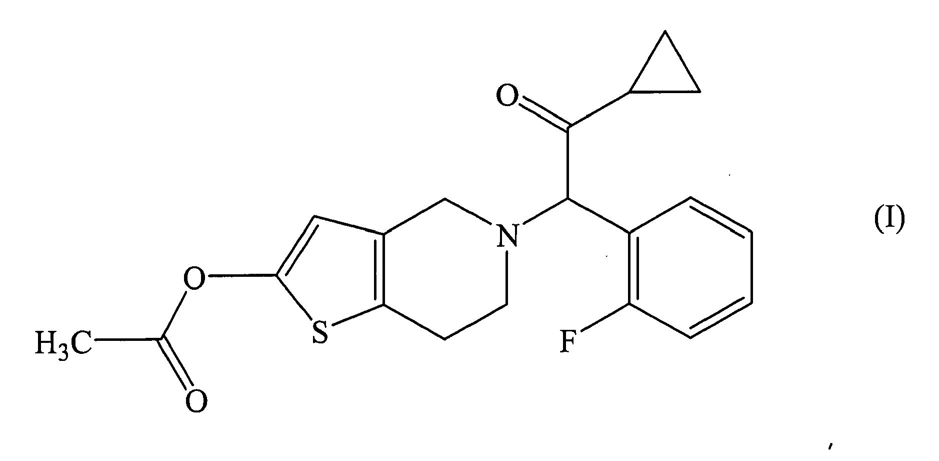 that is, 2-acetoxy-5-(α-cyclopropylcarbonyl-2-fluorobenzyl)-4,5,6,7-tetrahydrothieno[3,2-c]pyridine, or a pharmacologically acceptable salt thereof, which is the active ingredient of the pharmaceutical composition of the present invention, is disclosed in Japanese Patent Application (Kokai) No. Hei 6-41139 or Japanese Patent Application (Kokai) No. 2002-145883, and can be prepared accordingly.

As the "pharmacologically acceptable salt thereof" of the present invention, there may be mentioned for example, hydrohalides such as hydrofluoride, hydrochloride, hydrobromide or hydroiodide; inorganic acid salts such as nitrate, perchloric acid salt, sulfate or phosphate; lower-alkyl sulfonic acid salts such as methanesulfonate, trifluoromethanesulfonate or ethanesulfonate; arylsulfonic acid salts such as benzenesulfonate or p-toluenesulfonate; organic acid salts such as acetate, malate, fumarate, succinate, citrate, ascorbate, tartrate, oxalate or maleate; or amino acid salts such as glycine salt, lysine salt, arginine salt, ornithine salt, glutamic acid salt or aspartic acid salt. The preferred salts are hydrohalides or organic acid salts, more preferably hydrochloride or maleate, and most preferably hydrochloride.

As the "water-soluble polymer" of the present invention, there may be mentioned for example, cellulose derivatives such as hydroxypropylmethyl cellulose, methyl cellulose, hydroxypropyl cellulose or sodium carboxymethyl cellulose; synthetic polymers such as polyvinylpyrrolidone, aminoalkyl methacrylate copolymer, carboxyvinyl polymer, polyvinyl alcohol or polyethylene glycol; HA "Sankyo" (manufactured by Sankyo Co., Ltd.); gum Arabic; agar; gelatin; or sodium alginate. Of these, hydroxypropylmethyl cellulose, hydroxypropyl cellulose or polyvinylpyrrolidone is preferably used, hydroxypropylmethyl cellulose or hydroxypropyl cellulose is more preferably used, and hydroxypropyl cellulose is most preferably used. In the present invention, the aforementioned water-soluble polymers can be used alone, or two or more types can be used in combination.

The pharmaceutical composition of the present invention may further contain additives such as appropriate pharmacologically acceptable fillers, lubricants, binders, emulsifiers, stabilizers, corrigents and/or diluents.

As the "fillers" used, there may be mentioned for example, organic fillers including sugar derivatives such as lactose, sucrose, glucose, mannitol or sorbitol; starch derivatives such as corn starch, potato starch, α-starch or dextrin; cellulose derivatives such as crystalline cellulose; gum Arabic; dextran; or pullulan: or inorganic fillers including silicate derivatives such as light anhydrous silicic acid, synthetic aluminum silicate, calcium silicate or magnesium metasilicate aluminate; phosphates such as calcium hydrogenphosphate; carbonates such as calcium carbonate; or sulfates such as calcium sulfate. Of these, one or more fillers selected from cellulose derivatives and sugar derivatives are preferably used, one or more fillers selected from lactose, mannitol and crystalline cellulose are more preferably used, and lactose and/or crystalline cellulose are most preferably used.

As the "lubricants" used, there may be mentioned for example, stearic acid; stearic acid metal salts such as calcium stearate or magnesium stearate; talc; colloidal silica; waxes such as beeswax or spermaceti; boric acid; adipic acid; sulfates such as sodium sulfate; glycol; fumaric acid; sodium stearyl fumarate; sucrose fatty acid esters; sodium benzoate; D,L-leucine; lauryl sulfates such as sodium lauryl sulfate or magnesium lauryl sulfate; silicates such as silicic anhydride or hydrated silicate; or the aforementioned starch derivatives. Of these, stearic acid metal salts are preferably used.

As the "binders" used, there may be mentioned for example, the same compounds as the aforementioned fillers.

As the "emulsifiers" used, there may be mentioned for example, colloidal clays such as bentonite or beegum; metal hydroxides such as magnesium hydroxide or aluminum hydroxide; anionic surfactants such as sodium lauryl sulfate or calcium stearate; cationic surfactants such as benzalkonium chloride; or nonionic surfactants such as polyoxyethylene alkyl ether, polyoxyethylene sorbitan fatty acid ester or sucrose fatty acid ester.

As the "stabilizers" used, there may be mentioned for example, para-oxybenzoic acid esters such as methyl paraben or propyl paraben; alcohols such as chlorobutanol, benzyl alcohol or phenyl ethyl alcohol; benzalkonium chloride; phenols such as phenol or cresol; thimerosal; dehydroacetic acid; or sorbic acid.

As the "corrigents" used, there may be mentioned for example, sweeteners such as sodium saccharin or aspartame; acidulants such as citric acid, malic acid or tartaric acid; or flavorings such as menthol, lemon or orange.

Although there is no particular limitation as regards the amount of the compound represented by the aforementioned general formula (I) or a pharmacologically acceptable salt thereof formulated in the entirety of the pharmaceutical composition, it is preferable to formulate 1.0 to 30.0 % by weight (preferably 1.3 to 20.0 % by weight) with respect to the total weight of the pharmaceutical composition.

Although there is no particular limitation as regards the amount of additives formulated in the entirety of the pharmaceutical composition, it is preferable to formulate 1.0 to 40.0 % by weight (preferably 2.5 to 20.0 % by weight) of water-soluble polymers, 10.0 to 93.5 % by weight (preferably 44.0 to 90.0 % by weight) of fillers, 0.5 to 5.0 % by weight (preferably 0.5 to 3.0 % by weight) of lubricants, and 0.0 to 15.0 % by weight (preferably 2.5 to 10.0 % by weight) of binders with respect to the total weight of the pharmaceutical composition.

The pharmaceutical composition of the present invention is preferably in a solid dosage form, and there may be mentioned for example, tablets (including sublingual tablets and tablets that disintegrate in the mouth), capsules (including soft capsules and microcapsules), granules, fine granules, powders, pills, chewables or troches, preferably powders, fine granules, granules, capsules or tablets, and most preferably tablets.

As regards production methods for the preparation of the present invention, there may be used a general method described in publications such as "Powder Technology and Pharmaceutical Process (D. Chulia et al., Elservier Science Pub Co (December 1, 1993))". In particular, a dry method (for example, a dry granulation method or a direct compression method, preferably a direct compression method) is preferable.

The "direct compression method" is a method in which raw material powders are directly subjected to compression-molding to produce a preparation.

The "dry granulation method" is a method in which a preparation is produced using granules prepared by crushing and dividing by an appropriate method a compression-molded slug or sheet of raw material powders. These methods are described in publications such as "The Theory and Practice of Industrial Pharmacy (Third Edition) (Leon Lachman et al.: LEA & FEBIGER 1986)" and "Pharmaceutical Dosage Forms: Tablets volume 1 (Second Edition) (Herbert A. Lieberman et al.: MARCEL DEKKER INC. 1989)".

Granulation used here means an operation of forming granules having an almost uniform shape and size from a raw material in the form of powders, mass, solution, or molten liquid, and examples include granulation for forming a final product such as granules, powders or fine granules, and granulation for forming an intermediate product for the production of a tablet or a capsule.

The compression-molding process is a process in which a mass product of raw material powder is formed by applying pressure to the raw material powder using mechanical force, and examples include rotary tableting machines (manufactured by Kikusui Seisakusho Ltd., Hata Iron Works Co., Ltd., Sugawara Seiki Co., Ltd. and the like), and dry granulators such as a roller compactor, a roll granulator, and a Chilsonator (manufactured by Freund Corporation, Turbo Kogyo Co., Ltd., Kurimoto, Ltd., Matsubo Corporation, Nippon Granulator Co., Ltd., Fuji Paudal Co., Ltd. and the like).

The crushing and dividing process is a process in which the mass product formed in the compression-molding process is crushed by means of a knife or cutter into an appropriate size, and examples of apparatuses used include mills and particle size selectors such as a power mill, Fitzmill, Fiore, and Co-mill (manufactured by Fuji Paudal Co., Ltd., Tokuju Corporation, Powrex Corporation and the like).

The thus obtained granulated product is subjected to particle size regulation so as to have a desired particle diameter, and then a preparation in the form of powders, fine granules or granules is produced. These preparations can also be produced as capsules by packing them in a capsule, or can be produced as tablets by further adding disintegrants and/or lubricants if necessary and subjecting them to compression-molding by a tableting machine or the like. The operations of mixing and granulation are both widely used in the field of formulation techniques, and those skilled in the art can carry them out appropriately. In addition, tablets may be provided with at least one layer of a film-coating.

Coating is conducted by using a film-coating machine for example, and as the film coating base agent, there may be mentioned for example, sugar coating base agents, water-soluble film coating base agents, enteric film coating base agents or sustained release film coating base agents.

As the sugar coating base agents, saccharose is used, and it can be used in combination with one or more selected from talc, precipitated calcium carbonate, calcium phosphate, calcium sulfate, gelatin, gum Arabic, polyvinylpyrrolidone and pullulan.

As the water-soluble film coating base agents, there may be mentioned for example, cellulose derivatives such as hydroxypropyl cellulose, hydroxypropylmethyl cellulose, hydroxyethyl cellulose, methyl hydroxyethyl cellulose and sodium carboxymethyl cellulose; synthetic polymers such as polyvinyl acetal diethyl aminoacetate, aminoalkyl methacrylate copolymer and polyvinylpyrrolidone; and polysaccharides such as pullulan.

As the enteric film coating base agents, there may be mentioned for example, cellulose derivatives such as hydroxypropylmethyl cellulose phthalate, hydroxypropylmethyl cellulose acetate succinate, carboxymethylethyl cellulose or cellulose acetate phthalate; acrylic acid derivatives such as (meth)acrylic acid copolymer L, (meth)acrylic acid copolymer LD or (meth)acrylic acid copolymer S; or natural substances such as shellac.

As the sustained release film coating base agents, there may be mentioned for example, cellulose derivatives such as ethyl cellulose; or acrylic acid derivatives such as aminoalkyl methacrylate copolymer RS or ethyl acrylate-methyl methacrylate copolymer emulsion.

The aforementioned coating base agents may be used by combining two or more of them in an appropriate ratio. In addition, the coating base agents may, if necessary, further include additives such as appropriate pharmacologically acceptable plasticizers, fillers, lubricants, masking agents, colorants and/or antiseptics.

The plasticizers which may be used in the present invention are not particularly limited, and a person skilled in the art can select them appropriately. As for such plasticizers, there may be mentioned for example, propylene glycol, polyethylene glycol, polypropylene glycol, glycerin and sorbitol, glycerin triacetate, diethyl phthalate and triethyl citrate, lauric acid, sucrose, dextrose, sorbitol, triacetin, acetyl triethyl citrate, triethyl citrate, tributyl citrate or acetyl tributyl citrate.

As the masking agents which may be used in the present invention, there may be mentioned for example, titanium oxide.

As the colorants which may be used in the present invention, there may be mentioned for example, titanium oxide, iron oxide, red ferric oxide, yellow ferric oxide or yellow No. 5 aluminum lake talc.

As the antiseptics which may be used in the present invention, there may be mentioned for example, paraben.

The dosage amount of the compound represented by the aforementioned general formula (I) or a pharmacologically acceptable salt thereof, which is an active ingredient of the pharmaceutical composition of the present invention, may vary depending on various conditions such as the activity of the drug, symptoms, age or body weight of a patient. The daily dosage amount for an adult human has a lower limit of 0.01 mg (preferably 1 mg) and an upper limit of 200 mg (preferably 100 mg) in the case of oral administration.

### [Examples]

The present invention will be described in more detail with reference to the Examples and Test Example; however, the present invention shall not be limited to these.

Here, "Compound A" used in the Examples is the compound represented by the following formula (Ia): 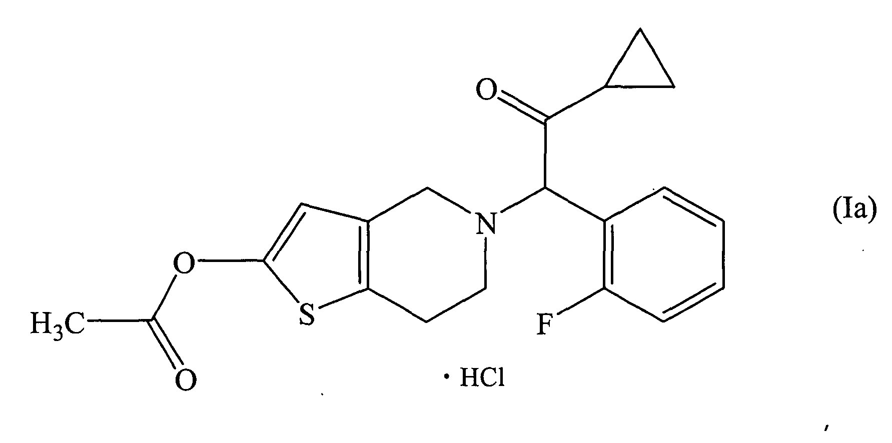 and can be prepared in accordance with the method disclosed in Japanese Patent Application (Kokai) No. 2002-145883.

### (Example 1)

Compound A (3.4 g), hydroxypropyl cellulose (17.5 g), croscarmellose sodium (12.5 g) and lactose (215.3 g) were mixed using a high intensity mixer for 3 minutes, followed by addition of magnesium stearate (1.3 g), and the mixture was mixed again using the high intensity mixer to give a mixed powder.

The mixed powder obtained was compressed using a rotary type tableting machine with a tableting pressure of 5.9 kN so that the tablet mass became approximately 80 mg. The uncoated tablet obtained was subjected to film-coating in a pan-coating machine, by spraying a coating solution consisting of hydroxypropylmethyl cellulose, lactose, titanium oxide, triacetin and water, to give a tablet containing the test compound. Stability testing was conducted on the obtained tablet. Test results are shown in Table 1.

### (Example 2)

Compound A (3.4 g), hydroxypropylmethyl cellulose (17.5 g), croscarmellose sodium (12.5 g) and lactose (215.3 g) were mixed using a high intensity mixer for 3 minutes, followed by addition of magnesium stearate (1.3 g), and the mixture was mixed again using the high intensity mixer to give a mixed powder.

The mixed powder obtained was compressed using a rotary type tableting machine with a tableting pressure of 5.9 kN so that the tablet mass became approximately 80 mg. The uncoated tablet obtained was subjected to film-coating in a pan-coating machine, by spraying a coating solution consisting of hydroxypropylmethyl cellulose, lactose, titanium oxide, triacetin and water, to give a tablet containing the test compound. Stability testing was conducted on the obtained tablet. Test results are shown in Table 1.

### (Example 3)

Compound A (3.4 g), polyvinylpyrrolidone (17.5 g), croscarmellose sodium (12.5 g) and lactose (215.3 g) were mixed using a high intensity mixer for 3 minutes, followed by addition of magnesium stearate (1.3 g), and the mixture was mixed again using the high intensity mixer to give a mixed powder.

The mixed powder obtained was compressed using a rotary type tableting machine with a tableting pressure of 5.9 kN so that the tablet mass became approximately 80 mg. The uncoated tablet obtained was subjected to film-coating in a pan-coating machine, by spraying a coating solution consisting of hydroxypropylmethyl cellulose, lactose, titanium oxide, triacetin and water, to give a tablet containing the test compound. Stability testing was conducted on the obtained tablet. Test results are shown in Table 1.

### (Comparative Example 1)

Compound A (3.4 g), croscarmellose sodium (12.5 g) and lactose (232.8 g) were mixed using a high intensity mixer for 3 minutes, followed by addition of magnesium stearate (1.3 g), and the mixture was mixed again using the high intensity mixer to give a mixed powder.

The mixed powder obtained was compressed using a rotary type tableting machine with a tableting pressure of 5.9 kN so that the tablet mass became approximately 80 mg. The uncoated tablet obtained was subjected to film-coating in a pan-coating machine, by spraying a coating solution consisting of hydroxypropylmethyl cellulose, lactose, titanium oxide, triacetin and water, to give a tablet containing the test compound. Stability testing was conducted on the obtained tablet. Test results are shown in Table 1.

### (Test Example 1) Stability Test

Tablets obtained in Examples 1 to 3 and Comparative Example 1 were each placed in an amber glass bottle, and were left to stand at 60°C under sealed conditions. After 3 weeks, the content of the active ingredient (the compound represented by the general formula (I)) in the test tablets was measured by high performance liquid chromatography. The measuring conditions for the high performance liquid chromatography were as follows.
Column: L-column ODS (4.6 mmID x 150 mm, manufactured by Chemicals Evaluation and Research Institute, Japan)
Mobile Phase: 0.01 mol/L phosphate buffer solution (pH 2.8)/acetonitrile mixture = 65/35 (v/v)
Column Temperature: constant temperature around 40°C
Wavelength detected: 260 nm

**(Table 1)**

| | Example 1 | Example 2 | Example 3 | Comparative Example 1 |
|---|---|---|---|---|
| Amount of active ingredient remaining (%) | 73 | 77 | 62 | 4 |

From Table 1, it is obvious that preparations containing a water-soluble polymer (Examples 1 to 3) have excellent storage stability compared with the preparation which does not contain a water-soluble polymer (Comparative Example).

### [INDUSTRIAL APPLICABILITY]

According to the present invention, a pharmaceutical composition having excellent storage stability, which contains the compound represented by the aforementioned general formula (I) or a pharmacologically acceptable salt thereof and a water-soluble polymer, can be obtained.

## Claims

1. A pharmaceutical composition comprising:
(A) a compound represented by the following general formula (I): 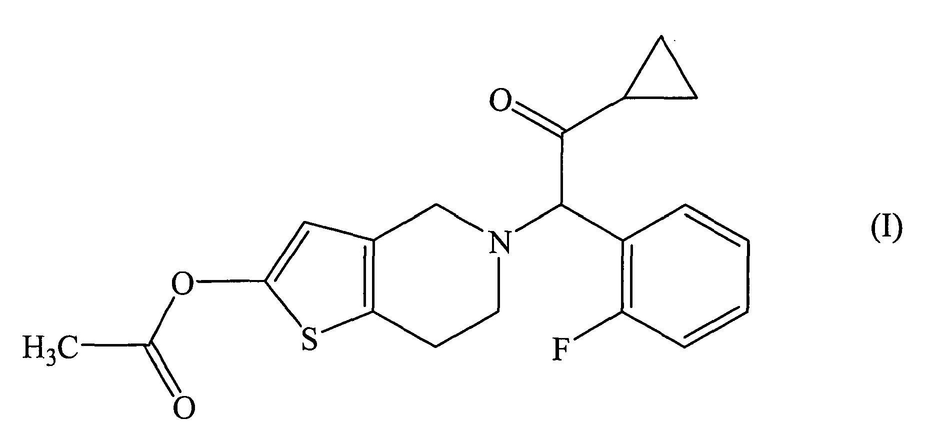 or a pharmacologically acceptable salt thereof; and
(B) a water-soluble polymer.

2. The pharmaceutical composition according to claim 1, wherein the water-soluble polymer is hydroxypropylmethyl cellulose, hydroxypropyl cellulose or polyvinylpyrrolidone.

3. The pharmaceutical composition according to claim 1, wherein the water-soluble polymer is hydroxypropylmethyl cellulose or hydroxypropyl cellulose.

4. The pharmaceutical composition according to claim 1, wherein the water-soluble polymer is hydroxypropyl cellulose.

5. The pharmaceutical composition according to any one of claims 1 to 4, wherein the amount of the water-soluble polymer formulated is 1.0 to 40.0% by weight with respect to the total amount of the pharmaceutical composition.

6. The pharmaceutical composition according to any one of claims 1 to 4, wherein the amount of the water-soluble polymer formulated is 2.5 to 20.0% by weight with respect to the total amount of the pharmaceutical composition.

7. The pharmaceutical composition according to any one of claims 1 to 6, wherein the compound represented by the general formula (I) or a pharmacologically acceptable salt thereof is a compound represented by the following formula (Ia):

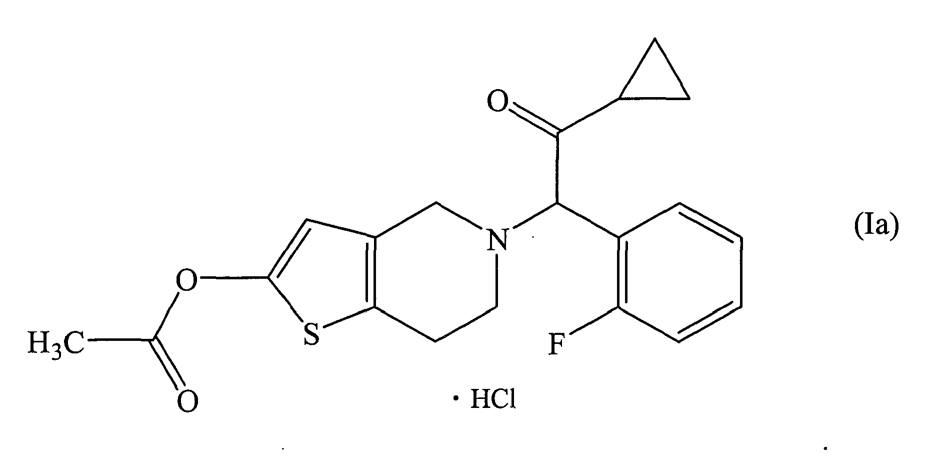

8. The pharmaceutical composition according to any one of claims 1 to 7, wherein the composition is in the form of powders, fine granules, granules, capsules or tablets.

9. The pharmaceutical composition according to any one of claims 1 to 7, wherein the composition is in the form of tablets.
